# EUROPEAN PATENT APPLICATION

(11) **EP 4 541 788 A1**
(43) Date of publication of application: **23.04.2025**
(21) Application number: 24203346.2
(22) Date of filing: 27.09.2024
(51) Int. Cl.: C07C 407/00, C07C 409/34

(54) **CONTINUOUS PRODUCTION OF AQUEOUS ORGANIC PEROXIDE EMULSIONS**

(30) Priority: 20.10.2023 EP 23205001
(71) Applicant: Nouryon Chemicals International B.V., 1101 BZ Amsterdam (NL)
(72) Inventor: GERRITSEN, René, 1101 BZ Amsterdam (NL); THAKURDESAI, Ameya Uday, 1101 BZ Amsterdam (NL)
(74) Representative: LKGlobal UK Ltd.

(57) **Abstract**

The present disclosure relates to a continuous process for producing aqueous organic peroxide emulsions comprising organic peroxides that have a low self-accelerating decomposition temperature, and a system for performing said continuous process.

## Description

### Technical Field

The present disclosure relates to a continuous process for producing aqueous organic peroxide emulsions comprising organic peroxides that have a low self-accelerating decomposition temperature, and a system for performing said continuous process.

### Background

There are many conventional methods in the technical field for producing aqueous organic peroxide emulsions. WO 99/31194, for example, explains that the emulsifying step may be carried out by using any conventional dispersing equipment, for example by means of a paddle-type, propeller-type, or turbine-type mechanical rotary stirrer, a colloid mill, a homogenizer, such as an ultra-sonic homogenizer, a high-speed shearing apparatus, and a line mixer.

Whilst these conventional options may be used for preparing aqueous emulsions of a wide range of temperature-stable organic peroxides, the currently available options are far more limited for organic peroxides that are less thermally stable. In that respect, for organic peroxides that have a low self-accelerating decomposition temperature ("SADT"; e.g., of about 25°C or less), the emulsification processes used to date have been batch processes. This is because the organic peroxide mixture must be maintained at a cool temperature (usually around or below 0°C) to prevent the organic peroxide from rapidly decomposing during the emulsification process, which can be extremely dangerous (e.g., can result in explosion). It was therefore considered too dangerous to attempt to produce aqueous emulsions of these peroxides in a continuous manner on an industrial scale.

US 6350835 discloses aqueous emulsions of peroxyesters that are liquid at -20°C. US 6350835 explains that the emulsification process is preferably performed at a temperature below the SADT of the peroxyester but fails to explain how the emulsification process could be performed in a safe, continuous manner; the worked examples of US 6350835 only provide conventional batch processes for producing aqueous emulsions of such peroxyesters.

US 4734135 discloses aqueous emulsions of peroxydicarbonates and diacylperoxides. Like US 6350835, the worked examples of US 4734135 only provide conventional batch-type processes for producing aqueous emulsions of such peroxides. Whilst US 4734135 speculates that the emulsion step "may be continuously carried out by an emulsifying device such as a line mixer, with a cooling device attached thereto" (i.e., a jacketed (cooling jacket) in-line mixer), this is neither exemplified in US 4734135, nor described in any further technical detail that would allow a person skilled in the technical field to reduce this to practice in a straightforward manner for any peroxide (and certainly not those with a low SADT).

The numerous benefits to continuous processes vs batch processes are well known. For example, continuous processes increase productivity and generally require a smaller equipment footprint. To the present inventors' knowledge, however, a successful, industrial scale, continuous process for producing aqueous emulsions of organic peroxides with a low SADT had not yet been developed.

It was therefore an objective of the present disclosure to develop a continuous process for producing aqueous emulsions of organic peroxides that have a low SADT. A further objective was to achieve this aim without the need to develop new specialist equipment.

### Description

The present inventors found that these objectives could be achieved using readily available equipment. In that respect, by cooling an organic peroxide-containing pre-emulsion mixture prior to being fed to an in-line emulsifier, followed by cooling the organic peroxide emulsion using a downstream heat exchanger, the present inventors have developed a somewhat simple, but very effective process and apparatus arrangement that solves the long felt need for a continuous process for producing aqueous emulsions of organic peroxides that have a low SADT.

Thus, in a first aspect, the present invention relates to a continuous process for producing an organic peroxide emulsion, the process comprising:
a) feeding a pre-emulsion mixture to an inlet of an in-line emulsifier, wherein the pre-emulsion mixture comprises at least one organic peroxide having a self-accelerating decomposition temperature (SADT) of 25°C or less, at least one protective colloid, at least one antifreeze agent, water, and optionally at least one emulsifier, and wherein the pre-emulsion mixture is fed to the inlet at a temperature of at least 12°C lower than the SADT of the organic peroxide, preferably at least 14°C lower than the SADT of the organic peroxide, more preferably from about 15°C to about 35°C lower than the SADT of the organic peroxide, and most preferably from about 15°C to about 25°C lower than the SADT of the organic peroxide, such as from about 15°C to about 20°C lower than the SADT of the organic peroxide; and
b) transferring an aqueous organic peroxide emulsion from an outlet of the in-line emulsifier to a heat exchanger positioned downstream of the outlet of the in-line emulsifier, wherein the heat exchanger reduces the temperature of the aqueous organic peroxide emulsion to a temperature of at least 12°C lower than the SADT of the organic peroxide, preferably at least 14°C lower than the SADT of the organic peroxide, more preferably from about 15°C to about 35°C lower than the SADT of the organic peroxide, and most preferably from about 15°C to about 25°C lower than the SADT of the organic peroxide, such as from about 15°C to about 20°C lower than the SADT of the organic peroxide.

In a second aspect, the present invention relates to a system for continuously producing an organic peroxide emulsion, the system comprising:
- at least one vessel for preparing and/or receiving a pre-emulsion mixture comprising at least one organic peroxide having a self-accelerating decomposition temperature (SADT) of 25°C or less, at least one protective colloid, at least one antifreeze agent, water, and optionally at least one emulsifier, wherein the at least one vessel comprises means for cooling and/or maintaining the pre-emulsion mixture at a temperature of at least 12°C lower than the SADT of the organic peroxide, preferably at least 14°C lower than the SADT of the organic peroxide, more preferably from about 15°C to about 35°C lower than the SADT of the organic peroxide, and most preferably from about 15°C to about 25°C lower than the SADT of the organic peroxide, such as from about 15°C to about 20°C lower than the SADT of the organic peroxide;
- an in-line emulsifier comprising at least one inlet and at least one outlet, wherein the in-line emulsifier is in fluid communication with and positioned downstream of the at least one vessel and configured to receive at the at least one inlet the pre-emulsion mixture at a temperature of at least 12°C lower than the SADT of the organic peroxide, preferably at least 14°C lower than the SADT of the organic peroxide, more preferably from about 15°C to about 35°C lower than the SADT of the organic peroxide, and most preferably from about 15°C to about 25°C lower than the SADT of the organic peroxide, such as from about 15°C to about 20°C lower than the SADT of the organic peroxide;

- a heat exchanger in fluid communication with and positioned downstream of the in-line emulsifier, wherein the heat exchanger is configured to reduce the temperature of an aqueous organic peroxide emulsion obtained from the at least one outlet of the in-line emulsifier to a temperature of at least 12°C lower than the SADT of the organic peroxide, preferably at least 14°C lower than the SADT of the organic peroxide, more preferably from about 15°C to about 35°C lower than the SADT of the organic peroxide, and most preferably from about 15°C to about 25°C lower than the SADT of the organic peroxide, such as from about 15°C to about 20°C lower than the SADT of the organic peroxide; and
- optionally a pack-out unit in fluid communication with and positioned downstream of the heat exchanger.

By using a specific feed temperature coupled with the introduction of a heat exchanger downstream of the outlet of the in-line emulsifier, the safety profile of the emulsification process is vastly improved to the extent that it can now be safely performed in a continuous and highly scalable manner (i.e., performed continuously on an industrial scale).

For the avoidance of any doubt, the "pre-emulsion mixture" is the mixture of components that has not been subjected to emulsifying conditions (and is thus not yet in the form of a stable emulsion).

The cooled aqueous organic peroxide emulsion obtained in step b) can be used directly (i.e., produced and used on-site) or it can be transferred to a pack-out unit positioned downstream of the heat exchanger (to be packaged and shipped). In that respect, the present invention provides for an easily installable system with a small equipment footprint for continuous on-site production of aqueous organic peroxide emulsions wherein the organic peroxide has a low SADT. This is a substantial improvement in terms of ease of production, cost, safety, and environmental impact (lower volume of raw material to transport).

The SADT is the lowest ambient temperature at which self-accelerating decomposition occurs with a product in the packaging as used for transport. The method for determining the SADT of an organic peroxide is set out in detail in the United Nations Manual of Tests and Criteria, Seventh Revised Edition (2019), Test Series H.4: Heat accumulation storage test, which said manual and test method is hereby incorporated by reference. This method determines the minimum constant air environment temperature at which thermally unstable substances undergo exothermic decomposition at conditions representative of the substance when packaged. The method is based on the Semenov theory of thermal explosion i.e. the main resistance to heat flow is considered to be at the vessel walls. The method can be used for the determination of the SADT of a substance in its packaging, including IBCs and small tanks (up to 2 m³). For the avoidance of doubt, the SADT values referred to herein are determined in accordance with the method set out in the above referenced UN Manual (Test Series H.4: Heat accumulation storage test) for organic peroxides in a high-density polyethylene (HDPE) packaging with a volume of 20-30 liters.

The emulsion contains at least one organic peroxide having a self-accelerating decomposition temperature (SADT) of 25°C or less, preferably 20°C or less, preferably 15°C or less, preferably 10°C or less, preferably 5°C or less, and may be about 0°C or less. Preferred organic peroxides include peroxyesters, peroxydicarbonates and diacyl peroxides. Preferred are peroxyesters and diacyl peroxides. Examples of preferred organic peroxides for use in accordance with the present invention are diisobutyryl peroxide (SADT 0°C), Di-n-propyl peroxydicarbonate (SADT 0°C), Diisopropyl peroxydicarbonate (SADT 5°C), Di-n-butyl peroxydicarbonate (SADT 0°C), Di-sec-butyl peroxydicarbonate (SADT 0°C), Di(2-ethoxyethyl) peroxydicarbonate (SADT 10°C), di(2-ethylhexyl) peroxydicarbonate (SADT 5°C), 1,3-di(prop-2,2-diyl)benzene bis(neodecanoyl)peroxide (SADT 10°C), 1,1,3,3-tetramethylbutyl peroxyneodecanoate (SADT 15°C), 3-hydroxy-1,1-dimethylbutyl peroxyneodecanoate (SADT 10°C); tert-amyl peroxyneodecanoate (SADT 20°C); Cumyl peroxyneodecanoate (SADT 10°C), di-n-butyryl peroxide (SADT 25°C); tert-butyl peroxyneodecanoate (SADT 20°C); 1,1 ,3,3-tetramethylbutyl peroxypivalate (SADT 20°C); tert-amyl peroxypivalate (SADT 25°C); tert-butyl peroxypivalate (SADT 25°C); di(3,5,5-trimethylhexanoyl) peroxide (SADT 25°C), or mixtures thereof.

The organic peroxide emulsion typically contains 25-70 wt% of organic peroxide, based on the total weight of the emulsion. Preferably, the amount of organic peroxide in the emulsion is 30-65 wt%, more preferably 35-60 wt%, most preferably 40-60 wt%. The organic peroxide may be introduced into the emulsification process disclosed herein in the form of a phlegmatized organic peroxide. In that respect, any appropriate phlegmatizer may be used to dilute and/or dissolve the peroxide, such as inert phlegmatizing solvents. Such inert phlegmatizing solvents are well known in the art and include, for instance, isododecane and mineral oils. For example, 3-hydroxy-1,1-dimethylbutyl peroxyneodecanoate (SADT 10°C) is commercially available in phlegmatized form from Nouryon^{®} as a 75% solution in odorless mineral spirits ("Trigonox^{®} 193-CH75").

The amount of water in the emulsion typically ranges from 20-70 wt%, more preferably 25-50 wt%, and most preferably 25-40 wt%, based on the total weight of the emulsion.

The emulsion contains at least one anti-freeze agent and may comprise a combination of two or more antifreeze agents. Any conventional anti-freeze agent can be used. Preferably, use is made of methanol, ethanol, isopropanol, (ethylene) glycol, propanediol, glycerol, and mixtures thereof, more preferably, methanol, ethanol, propanediol, and mixtures thereof. These agents are known to have hardly any effect on polymerization processes. A skilled person will have no difficulties in balancing the ratio of water to anti-freeze agent(s). Typically, the amount of anti-freeze agent used in the emulsion according to the present invention will be lower than the amount of water.

The emulsion also contains at least one protective colloid. Any conventional protective colloid can be used. Suitable protective colloids include partially hydrolyzed (or saponified) polyvinyl acetates, polyvinyl pyrrolidones, polyacrylates, cellulose, cellulose derivatives, starch, and starch derivatives. Particularly useful are partially hydrolyzed/saponified polyvinyl acetates, cellulose, cellulose derivatives, starch, and starch derivatives. Typically, a polyvinyl acetate (PVA) is used, preferably a PVA having a degree of hydrolysis of 50-90 mole%, more preferably 55-80 mole%, more preferably 60-70 mole%. The amount of protective colloid used in the emulsions according to the present invention will depend on the type and amount of organic peroxide and the desired viscosity of the final emulsion, but will typically be used in an amount of from about 0.1 to about 5 wt.%, based on the total weight of the emulsion.

The emulsion in accordance with the present invention may contain at least one conventional emulsifying agent. Suitable emulsifying agents are known to the person skilled in this art and they include non-ionic, anionic, cationic, and amphoteric surfactants, and mixtures thereof. They may be incorporated in their usual amounts, such as from about 0.05 to about 4 wt.%, based on the total weight of the emulsion. Preferably, a non-ionic surfactant, more preferably having an HLB (hydrophile-lipophile balance) value of 7 or higher, even more preferably 16 or higher, is used. Alternatively, the emulsion in accordance with the present invention may contain very little amounts of emulsifier, or none at all. It has been found that substantially or completely removing the emulsifier - most notably emulsifiers in the form of non-ionic, anionic, cationic, and amphoteric surfactants - from the peroxide emulsion is highly beneficial in the production of polyvinylchloride (PVC). In that respect, such "emulsifier-free" emulsions of low SADT peroxides (particularly diisobutyryl peroxide, SADT 0°C) have been found to produce high-quality PVC from vinyl chloride monomer (VCM) with unexpected improvements in drying properties of the polymer (the drying rate of the resulting PVC polymer was substantially increased, thus shortening the drying time of the polymer, which is important for the industrial-scale production of PVC). Accordingly, in a preferred embodiment, the emulsion in accordance with the present invention may contain emulsifiers in a total amount of 0 wt.% to 0.17 wt.%, based on the total weight of the emulsion. Preferably still, the emulsion in accordance with the present invention may contain 0 wt.% to 0.17 wt.%, more preferably 0 wt.%, of (non-ionic) surfactants having an HLB (hydrophile-lipophile balance) value of 7 or higher (wt.% based on the total weight of the emulsion). Thus, the emulsion in accordance with the present invention may contain emulsifiers in a total amount of 0 wt.% to about 4 wt.%, based on the total weight of the emulsion.

The aqueous organic peroxide emulsions of the present invention optionally may also contain other additives including pH-adjusting agents such as phosphate and citrate buffers, sequestering agents, biocides, e.g. fungicides, antiozonants, antioxidants, anti-degradants, U.V. stabilizers, coagents, comonomers, antistatic agents, blowing agents, mould release agents, and process oils. These additives may be added in their usual amounts.

The process and system of the present disclosure requires an in-line emulsifier. In-line emulsifiers suitable for use in the present process are very well-known in the art, such as (but not limited to) those discussed in WO 99/31194 and described in US 2004/223407, and suitable in-line emulsifiers are widely available, such as (but not limited to) the Process Pilot 2000, the DISPAX-REACTOR^{®} DRS 2000, and various configurations and scale up equivalents thereof that are commercially available from IKAO-WERKE GmbH & Co. KG. These well-known in-line emulsifiers are typically not used at low (e.g., sub-zero) working temperatures, however it is straightforward to make routine modifications to those emulsifiers to accommodate the low temperatures (e.g., by changing the material of replaceable components, such as O-rings, to those that are known to be mechanically stable at very low temperatures). Such routine modifications would be well within the routine capabilities of a person skilled in the technical field.

Preferably, the residence time of the organic peroxide mixture in the in-line emulsifier is between about 1 and about 10 seconds, preferably between about 2 and about 8 seconds, and more preferably between about 3 and about 6 seconds, such as from about 3.8 to about 5.5 seconds.

The in-line emulsifier preferably comprises at least one mill head, preferably at least two mill heads, and may comprise at least three mill heads. The in-line emulsifier may comprise the mill head(s) in a rotor-stator arrangement and may be configured as a single stage rotor-stator system or may be configured as a multiple (e.g., two or three) stage rotor-stator system. The mill head(s) may be of the coarse type, medium type, fine type, or combinations thereof. In a preferred embodiment, the tip speed of the or each mill head is from about 15 metres/second (m/s) to about 50 m/s, preferably from about 20 m/s to about 45 m/s, and more preferably from about 25 m/s to about 40 m/s.

It would be well within the routine capabilities of a skilled person to configure the in-line emulsifiers known in the art to work within these operational parameters.

Thus, in a preferred embodiment, the continuous process for producing an organic peroxide emulsion comprises:
a) feeding a pre-emulsion mixture to an inlet of an in-line emulsifier, wherein the in-line emulsifier comprises at least one mill head, preferably at least two mill heads, wherein the tip speed of the or each mill head is from 15 m/s to 50 m/s, preferably from about 20 m/s to about 45 m/s, and more preferably from about 25 m/s to about 40 m/s, wherein the pre-emulsion mixture comprises at least one organic peroxide having a self-accelerating decomposition temperature (SADT) of 25°C or less, at least one protective colloid, at least one antifreeze agent, water, and optionally at least one emulsifier, and wherein the pre-emulsion mixture is fed to the inlet at a temperature of at least 12°C lower than the SADT of the organic peroxide, preferably at least 14°C lower than the SADT of the organic peroxide, more preferably from about 15°C to about 35°C lower than the SADT of the organic peroxide, and most preferably from about 15°C to about 25°C lower than the SADT of the organic peroxide, such as from about 15°C to about 20°C lower than the SADT of the organic peroxide; and
b) transferring an aqueous organic peroxide emulsion from an outlet of the in-line emulsifier to a heat exchanger positioned downstream of the outlet of the in-line emulsifier, wherein the heat exchanger reduces the temperature of the aqueous organic peroxide emulsion to a temperature of at least 12°C lower than the SADT of the organic peroxide, preferably at least 14°C lower than the SADT of the organic peroxide, more preferably from about 15°C to about 35°C lower than the SADT of the organic peroxide, and most preferably from about 15°C to about 25°C lower than the SADT of the organic peroxide, such as from about 15°C to about 20°C lower than the SADT of the organic peroxide,
wherein the residence time of the pre-emulsion mixture in the in-line emulsifier is between about 1 and about 10 seconds, preferably between about 2 and about 8 seconds, and more preferably between about 3 and about 6 seconds, such as from about 3.8 to about 5.5 seconds.

In a preferred embodiment, the system for continuously producing an organic peroxide emulsion comprises:
- at least one vessel for preparing and/or receiving a pre-emulsion mixture (e.g., at least one (continuous) stirred tank reactor) comprising at least one organic peroxide having a self-accelerating decomposition temperature (SADT) of 25°C or less, at least one protective colloid, at least one antifreeze agent, water, and optionally at least one emulsifier, wherein the at least one vessel comprises means for cooling and/or maintaining the pre-emulsion mixture (e.g., internal and/or external cooling coil, external cooling jacket, etc.) at a temperature of at least 12°C lower than the SADT of the organic peroxide, preferably at least 14°C lower than the SADT of the organic peroxide, more preferably from about 15°C to about 35°C lower than the SADT of the organic peroxide, and most preferably from about 15°C to about 25°C lower than the SADT of the organic peroxide, such as from about 15°C to about 20°C lower than the SADT of the organic peroxide;
- an in-line emulsifier comprising at least one inlet and at least one outlet, wherein the in-line emulsifier comprises at least one mill head, preferably at least two mill heads, wherein the or each mill head is configured to have a tip speed of from 15 m/s to 50 m/s, preferably from about 20 m/s to about 45 m/s, and more preferably from about 25 m/s to about 40 m/s, wherein the in-line emulsifier is in fluid communication with and positioned downstream of the at least one vessel and configured to receive at the at least one inlet the pre-emulsion mixture at a temperature of at least 12°C lower than the SADT of the organic peroxide, preferably at least 14°C lower than the SADT of the organic peroxide, more preferably from about 15°C to about 35°C lower than the SADT of the organic peroxide, and most preferably from about 15°C to about 25°C lower than the SADT of the organic peroxide, such as from about 15°C to about 20°C lower than the SADT of the organic peroxide;
- a heat exchanger in fluid communication with and positioned downstream of the in-line emulsifier, wherein the heat exchanger is configured to reduce the temperature of an aqueous organic peroxide emulsion obtained from the at least one outlet of the in-line emulsifier to a temperature of at least 12°C lower than the SADT of the organic peroxide, preferably at least 14°C lower than the SADT of the organic peroxide, more preferably from about 15°C to about 35°C lower than the SADT of the organic peroxide, and most preferably from about 15°C to about 25°C lower than the SADT of the organic peroxide, such as from about 15°C to about 20°C lower than the SADT of the organic peroxide; and
- optionally, a pack-out unit positioned downstream of the heat exchanger;
wherein the system is configured such that the residence time of the pre-emulsion mixture in the in-line emulsifier is between about 1 and about 10 seconds, preferably between about 2 and about 8 seconds, and more preferably between about 3 and about 6 seconds, such as from about 3.8 to about 5.5 seconds.

In a preferred embodiment, the system further comprises at least one mixing apparatus in fluid communication with and positioned upstream of the at least one vessel for preparing and/or receiving a pre-emulsion mixture. In said embodiment, the at least one mixing apparatus prepares a mixture comprising the protective colloid, the antifreeze agent, water, and the optional emulsifier. Said mixture is then transferred to the at least one vessel wherein it is mixed with the at least one organic peroxide having a SADT of 25°C or less to form the pre-emulsion mixture. In one embodiment, the mixing apparatus may be a continuous stirred tank reactor (CSTR). In another embodiment, the mixing apparatus may be an inline static mixer comprising one or more static mixer elements. Such mixers are well known to persons skilled in the technical field.

Accordingly, in a preferred embodiment the present invention relates to a system for continuously producing an organic peroxide emulsion, the system comprising:
- at least one mixing apparatus for preparing a mixture comprising at least one protective colloid, at least one antifreeze agent, water, and optionally at least one emulsifier;
- at least one vessel in fluid communication with and positioned downstream of the at least one mixing apparatus and configured to receive the mixture comprising at least one protective colloid, at least one antifreeze agent, water, and optionally at least one emulsifier, the at least one vessel being further configured to separately receive at least one organic peroxide having a self-accelerating decomposition temperature (SADT) of 25°C or less and to prepare a pre-emulsion mixture comprising the at least one organic peroxide having a self-accelerating decomposition temperature (SADT) of 25°C or less, at least one protective colloid, at least one antifreeze agent, water, and optional at least one emulsifier, wherein the at least one vessel comprises means for cooling and/or maintaining the pre-emulsion mixture at a temperature of at least 12°C lower than the SADT of the organic peroxide, preferably at least 14°C lower than the SADT of the organic peroxide, more preferably from about 15°C to about 35°C lower than the SADT of the organic peroxide, and most preferably from about 15°C to about 25°C lower than the SADT of the organic peroxide, such as from about 15°C to about 20°C lower than the SADT of the organic peroxide;
- an in-line emulsifier comprising at least one inlet and at least one outlet, wherein the in-line emulsifier is in fluid communication with and positioned downstream of the at least one vessel and configured to receive at the at least one inlet the pre-emulsion mixture at a temperature of at least 12°C lower than the SADT of the organic peroxide, preferably at least 14°C lower than the SADT of the organic peroxide, more preferably from about 15°C to about 35°C lower than the SADT of the organic peroxide, and most preferably from about 15°C to about 25°C lower than the SADT of the organic peroxide, such as from about 15°C to about 20°C lower than the SADT of the organic peroxide;
- a heat exchanger in fluid communication with and positioned downstream of the in-line emulsifier, wherein the heat exchanger is configured to reduce the temperature of an aqueous organic peroxide emulsion obtained from the at least one outlet of the in-line emulsifier to a temperature of at least 12°C lower than the SADT of the organic peroxide, preferably at least 14°C lower than the SADT of the organic peroxide, more preferably from about 15°C to about 35°C lower than the SADT of the organic peroxide, and most preferably from about 15°C to about 25°C lower than the SADT of the organic peroxide, such as from about 15°C to about 20°C lower than the SADT of the organic peroxide; and
- optionally a pack-out unit in fluid communication with and positioned downstream of the heat exchanger.

It is noted that various elements of the present invention, including but not limited to preferred ranges for the various parameters, can be combined unless they are mutually exclusive.

The invention will be elucidated by the following Figures and Examples without being limited thereto or thereby.

### Figures

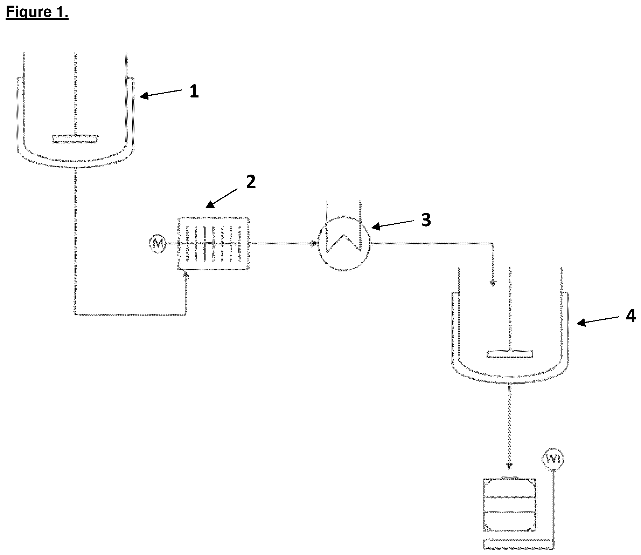

Figure 1 is a schematic representation of a system in accordance with the present invention.

### Worked Example

With reference to Figure 1, a continuous process was developed for the continuous production of an aqueous emulsion of diisobutyryl peroxide (SADT 0°C).

A pre-emulsion mixture comprising methanol (15 wt.%), polyvinyl acetate having a degree of hydrolysis of 50-75 mole% (1.5 wt.%), nonionic surfactant (1 wt.%), diisobutyryl peroxide (40 wt.%) and water was cooled to -15°C with stirring in vessel **1.**

The cooled pre-emulsion mixture from vessel **1** was fed continuously to the inlet of the in-line emulsifier **2** at a temperature of -15°C. The in-line emulsifier delivered shear force to the pre-emulsion mixture to form a stable emulsion of diisobutyryl peroxide. The emulsified product was discharged from the in-line emulsifier outlet and transferred to the heat exchanger **3,** where the emulsified product was cooled to -15 °C. Thereafter, the product was fed to pack-out buffer tank **4** and packed out from the packing lines downstream.

No safety concerns were noted at any point during the continuous emulsification process.

### Description of apparatus features:

***1.*** *Vessel equipped with stirrer and external cooling coils configured to cool the pre-emulsion mixture contained therein to -15°C*
***2**. DISPAX-REACTOR^{®} type DRS 2000*/*10 equipped with at least 2 mill heads each operating at a tip speed of about 40 m*/*s and having a peroxide residence time of between 3.8 seconds to 5.5 seconds.*
***3**. Heat exchanger configured to reduce the temperature of the organic peroxide emulsion to -15°C*
***4**. Pack out unit (buffer tank* & *downstream packaging vessel)*

In this specification, unless expressly otherwise indicated, the word 'or' is used in the sense of an operator that returns a true value when either or both of the stated conditions is met, as opposed to the operator 'exclusive or' which requires that only one of the conditions is met. The word 'comprising' is used in the sense of 'including' rather than to mean 'consisting of'. All prior teachings acknowledged above are hereby incorporated by reference. No acknowledgement of any prior published document herein should be taken to be an admission or representation that the teaching thereof was common general knowledge in Europe or elsewhere at the date hereof.

## Claims

1. A continuous process for producing an organic peroxide emulsion, the process comprising:
a) feeding a pre-emulsion mixture to an inlet of an in-line emulsifier, wherein the pre-emulsion mixture comprises at least one organic peroxide having a self-accelerating decomposition temperature (SADT) of 25°C or less, at least one protective colloid, at least one antifreeze agent, water, and optionally at least one emulsifier, and wherein the pre-emulsion mixture is fed to the inlet at a temperature of at least 12°C lower than the SADT of the organic peroxide;
b) transferring an aqueous organic peroxide emulsion from an outlet of the in-line emulsifier to a heat exchanger downstream of the outlet of the in-line emulsifier, wherein the heat exchanger reduces the temperature of the aqueous organic peroxide emulsion to a temperature of at least 12°C lower than the SADT of the organic peroxide.

2. The process of claim 1, wherein the pre-emulsion mixture of step a) is fed to the inlet at a temperature of at least 14°C lower than the SADT of the organic peroxide, preferably from about 15°C to about 35°C lower than the SADT of the organic peroxide, and most preferably from about 15°C to about 25°C lower than the SADT of the organic peroxide, such as from about 15°C to about 20°C lower than the SADT of the organic peroxide.

3. The process of any one of the preceding claims, wherein in step b) the heat exchanger reduces the temperature of the organic peroxide emulsion to a temperature of at least 14°C lower than the SADT of the organic peroxide, preferably from about 15°C to about 35°C lower than the SADT of the organic peroxide, and most preferably from about 15°C to about 25°C lower than the SADT of the organic peroxide, such as from about 15°C to about 20°C lower than the SADT of the organic peroxide.

4. The process of any one of the preceding claims, further comprising c) transferring the cooled aqueous organic peroxide emulsion of step b) to a pack-out unit positioned downstream of the heat exchanger.

5. The process of any one of the preceding claims, wherein the organic peroxide has a self-accelerating decomposition temperature (SADT) of 20°C or less, preferably 15°C or less, preferably 10°C or less, preferably 5°C or less.

6. The process of any one of the preceding claims, wherein the organic peroxide is selected from peroxyesters, peroxydicarbonates, diacyl peroxides, or mixtures thereof.

7. The process of any one of the preceding claims, wherein the organic peroxide is selected from diisobutyryl peroxide (SADT 0°C), Di-n-propyl peroxydicarbonate (SADT 0°C), Diisopropyl peroxydicarbonate (SADT 5°C), Di-n-butyl peroxydicarbonate (SADT 0°C), Di-sec-butyl peroxydicarbonate (SADT 0°C), Di(2-ethoxyethyl) peroxydicarbonate (SADT 10°C), di(2-ethylhexyl) peroxydicarbonate (SADT 5°C), 1,3-di(prop-2,2-diyl)benzene bis(neodecanoyl)peroxide (SADT 10°C), 1,1,3,3-tetramethylbutyl peroxyneodecanoate (SADT 15°C), 3-hydroxy-1,1-dimethylbutyl peroxyneodecanoate (SADT 10°C); tert-amyl peroxyneodecanoate (SADT 20°C); Cumyl peroxyneodecanoate (SADT 10°C), di-n-butyryl peroxide (SADT 25°C); tert-butyl peroxyneodecanoate (SADT 20°C); 1,1,3,3-tetramethylbutyl peroxypivalate (SADT 20°C); tert-amyl peroxypivalate (SADT 25°C); tert-butyl peroxypivalate (SADT 25°C); di(3,5,5-trimethylhexanoyl) peroxide (SADT 25°C), or mixtures thereof.

8. The process of any one of the preceding claims, wherein the pre-emulsion mixture comprises 25-70 wt% of organic peroxide, based on the total weight of the pre-emulsion mixture.

9. The process of any one of the preceding claims, wherein the at least one protective colloid is selected from partially hydrolyzed (or saponified) polyvinyl acetates, polyvinyl pyrrolidones, polyacrylates, cellulose, cellulose derivatives, starch, and starch derivatives, preferably partially hydrolyzed (or saponified) polyvinyl acetates.

10. The process of any one of the preceding claims, wherein the at least one emulsifier is omitted from the pre-emulsion mixture, or is selected from non-ionic, anionic, cationic, and amphoteric surfactants, and mixtures thereof, preferably a non-ionic surfactant.

11. The process of any one of the preceding claims, wherein the at least one antifreeze agent is selected from methanol, ethanol, isopropanol, (ethylene) glycol, propanediol, glycerol, and mixtures thereof.

12. The process of any one of the preceding claims, wherein the in-line emulsifier comprises at least one mill head, preferably at least two mill heads, and wherein the tip speed of the or each mill head is from about 15 metres/second (m/s) to about 50 m/s, preferably from about 20 m/s to about 45 m/s, and more preferably from about 25 m/s to about 40 m/s.

13. The process of any one of the preceding claims, wherein the residence time of the pre-emulsion mixture in the in-line emulsifier is between about 1 and about 10 seconds, preferably between about 2 and about 8 seconds, and more preferably between about 3 and about 6 seconds, such as from about 3.8 to about 5.5 seconds.

14. A system for continuously producing an organic peroxide emulsion, the system comprising:
• at least one vessel for preparing and/or receiving a pre-emulsion mixture comprising at least one organic peroxide having a self-accelerating decomposition temperature (SADT) of 25°C or less, at least one protective colloid, at least one antifreeze agent, water, and optionally at least one emulsifier, wherein the at least one vessel comprises means for cooling and/or maintaining the pre-emulsion mixture at a temperature of at least 12°C lower than the SADT of the organic peroxide;
• an in-line emulsifier comprising at least one inlet and at least one outlet, wherein the in-line emulsifier is in fluid communication with and positioned downstream of the at least one vessel and configured to receive at the at least one inlet the pre-emulsion mixture at a temperature of at least 12°C lower than the SADT of the organic peroxide; and
• a heat exchanger in fluid communication with and positioned downstream of the in-line emulsifier, wherein the heat exchanger is configured to reduce the temperature of an aqueous organic peroxide emulsion obtained from the at least one outlet of the in-line emulsifier to a temperature of at least 12°C lower than the SADT of the organic peroxide.

15. The system of claim 14, further comprising a pack-out unit positioned downstream of the heat exchanger.
